# EUROPÄISCHE PATENTSCHRIFT

(11) **EP 2 345 389 B1**
(45) Veröffentlichungstag und Bekanntmachung des Hinweises auf die Patenterteilung: **06.08.2014**
(21) Anmeldenummer: 10196738.8
(22) Anmeldetag: 23.12.2010
(51) Int. Cl.: A61F 2/08, A61B 17/88

(54) **Einbringinstrument zum Einschlagen eines Verblockungselementes**
Insertion instrument for hammering a blocking element
Instrument d'introduction pour enfoncer un élément de blocage

(30) Priorität: 18.01.2010 DE 102010005659
(43) Veröffentlichungstag der Anmeldung: 20.07.2011
(73) Patentinhaber: Karl Storz GmbH & Co. KG, 78532 Tuttlingen (DE)
(72) Erfinder: Friedrich, Markus, 78628, Rottweil (DE); Berberich, Sascha, 78532, Tuttlingen (DE); Frey, Sebastian, 78054, Villingen-Schwenningen (DE); Sauer, Michael, 78532 Tuttlingen (DE); Bogenschütz, Jürgen, 88499 Altheim (DE)
(74) Vertreter: Witte, Weller & Partner Patentanwälte mbB

(56) Entgegenhaltungen:
- EP-A2- 1 234 545
- WO-A1-2005/122970
- US-A1- 2007 270 973
- US-A1- 2008 109 006

## Beschreibung

Die Erfindung betrifft ein Einbringinstrument zum Einschlagen eines Verblockungselementes in einen Bohrkanal zum Fixieren eines Sehnenersatzes in dem Bohrkanal bei einer Rekonstruktion eines Kreuzbandes eines Kniegelenkes.

Ein derartiges Instrument ist aus der EP 1 234 545 A2 bekannt.

Bei der Rekonstruktion des vorderen Kreuzbandes eines Kniegelenkes wird meist, wie das aus der US 2009/0018654 A1 beschrieben ist, so vorgegangen, dass in der Tibia (dem Unterschenkelknochen) und dem Femur (dem Oberschenkelknochen) jeweils von der Außenseite her in Richtung Mitte des Gelenkes ein Kanal gebohrt wird.

In die beiden Kanäle wird dann ein Sehnenersatz eingelegt, der fixiert werden muss.

Dazu ist u.a. bekannt geworden, in den Bohrkanal in einen Zwischenraum zwischen der Innenseite des Bohrkanals und dem darin eingesetzten Sehnenimplantat ein Verblockungselement einzusetzen.

Mit dem Verblockungselement soll der Sehnenersatz unverschiebbar in dem jeweiligen Bohrkanal fixiert werden.

Es ist bekannt geworden, als Verblockungselement sogenannte Interferenzschrauben einzusetzen. Dies sind kopflose Schrauben mit einem relativ groben, jedoch abgerundeten Gewinde.

Diese Interferenzschrauben werden meist von der Außenseite her über ein Einbringinstrument, meist einen Schraubendreher, in den Bohrkanal eingedreht, um dadurch das Sehnenimplantat in dem Bohrkanal zu fixieren.

Grundsätzlich nachteilig an einer Interferenzschraube ist, dass diese beim Eintreiben gedreht werden muss, so dass nicht ausgeschlossen werden kann, dass auch das Implantat, das durch die Interferenzschraube an die Innenwand der Bohrung gepresst oder gequetscht werden soll, etwas verdrillt.

Ist das Sehnenimplantat eine natürliche andere Sehne des Körpers und wird beim Fixieren diese Sehne beschädigt, besteht die Gefahr, dass das Sehnenimplantat bei der nachfolgenden Belastung reißt oder ein Anwachsen des verletzten Sehnenimplantates in dem Bohrkanal verzögert oder gar verhindert wird.

Ferner hat sich herausgestellt, dass es günstig ist, eine gelenknahe Fixierung des Sehnenersatzes in dem Bohrkanal zu bewerkstelligen. Darunter ist zu verstehen, dass das Verblockungselement meist von außen (extraartikulär) so weit in beispielsweise den tibialen Bohrkanal eingeschraubt wird, bis die Schraubenspitze in der Nähe des Tibiaplateaus liegt. Diese gelenknahe Fixierung soll verhindern, dass ein Endabschnitt des Bohrkanals zwischen Verblockungselement und der Knieinnenseite nur durch den Sehnenersatz belegt ist. Bei Belastung, also beim Beugen von Ober-und Unterschenkel, beispielsweise beim Übergang vom Sitzen in Stehen oder umgekehrt, wird der Sehnensersatz in dem Endabschnitt seitlich in dem Bohrkanal hin und her bewegt, und weitet diesen dabei trompetenartig auf. Dies verhindert ein schnelles Ein- und Anwachsen des Sehnenersatzes, was unerwünscht ist. Daher wird die gelenknahe Fixierung angestrebt.

Wird eine Interferenzschraube nun von außen (extraartikulär) durch einen relativ langen tibialen oder femoralen Bohrkanal in Richtung Knieinnenseite vorgetrieben, so muss sie mehrfach gedreht werden, was die eingangs erwähnten Verletzungen an dem Sehnenersatz nach sich ziehen kann.

Aus der US 2009/0265004 A1 ist eine Fixationstechnik bekannt geworden, bei der eine Interferenzschraube über einen flexiblen Faden von Seiten des Tibiaplateaus in einen tibialen Bohrkanal durch das Kniegelenk hindurch eingezogen und dann durch ein von der Außenseite her angesetztes Werkzeug eingedreht werden kann. Hier kann eine gelenknahe Fixation durch wenige Drehvorgänge der Schraube bewerkstelligt werden, dennoch sind Drehvorgänge notwendig und die Interferenzschraube muss durch ein Zugelement zwischen Tibia und Femur eingebracht und in den tibialen Bohrkanal eingezogen werden.

Aus der DE 10 2004 042 183 A1 ist ein Gerät zum Setzen oder Entfernen von Gelenken oder Gelenkpfannen bekannt. Das Gerät weist eine Handhabe, eine Gelenkpfannen-Aufnahme oder -Halterung und ein zwischen Handhabe und Gelenkpfannenhalterung angeordnetes Verbindungselement auf, wobei das Verbindungselement in Richtung einer Längserstreckung einen bogenförmigen Abschnitt umfasst. Das proximale Ende ist mit einem Handgriff versehen, dessen äußeres proximales Ende einen Kopf aufweist, der als Schlagkopf dienen kann. Ein Schlagen auf den Schlagkopf bewirkt ein Eintreiben der Pfanne in eine vorbereitete Stelle am Knochen.

In der eingangs erwähnten EP 1 234 545 A2 ist ein Einbringinstrument zum Einschlagen eines Verblockungselementes in einem Bohrkanal zum Fixieren eines Sehnenersatzes in dem Bohrkanal bei einer Rekonstruktion eines Kreuzbandes eines Kniegelenkes beschrieben. Ein Griff zum Ergreifen des Einbringelementes ist an seinem proximalen Ende mit einem Schlagkopf versehen. Vom distalen Ende des Griffes erstreckt sich seitlich etwa im rechten Winkel ein Bügel fort, an dessen äußerem Ende eine Öffnung vorhanden ist. In diese Öffnung ist längsverschieblich und drehbar ein stabförmiger Körper eingesetzt. Die beiden Bauteile, Bügel und Stab, stehen etwa in einem rechten Winkel zueinander.

Am distalen Ende des Stabes ist ein abgewinkelter Abschnitt vorhanden, an dessen distalem Ende das Verblockungselement angesetzt werden kann. Die Abwinklung ist so, dass sich die Längsachse des Verblockungselementes in der Längsachse des Griffes erstreckt.

Somit können Kräfte, die in der Schlaglinie auf den Handgriff aufgeübt werden, über den seitlich abstehenden Bügel und den darin aufgenommenen Stab auf das Einschlagelement übertragen werden.

Aus der US 2008/0109006 A1 ist ein Einschläger für ein Werkzeug bekannt, der am proximalen Ende einen Handgriff mit einem Schlagkopf und am distalen Ende einen Abschnitt aufweist, von dessen Längserstreckung sich ein Verbindungsstück für ein Werkzeug weg erstreckt.

Zwischen diesen endseitigen Bauelementen sind mehrere gelenkig zueinander über Schwenkzapfen verbundene Laschen vorhanden. Dadurch ist es möglich, zwischen den beiden endseitigen Abschnitten den Körper nicht nur geradlinig erstreckt auszurichten, sondern entsprechend um die einzelnen linearen Laschen seitlich verschwenkt verlaufend auszurichten. In allen dargestellten Verschwenkmöglichkeiten dieser Zwischenkörper, verlaufen das lineare proximale Endstück mit dem Schlagkopf und das lineare distale Endstück in einer Linie ausgerichtet zueinander, die auch zugleich die Schlaglinie ist.

Aus der US 2007/0270973 A1 ist eine Vorrichtung zum Platzieren eines Hüftgelenkimplantates in einem Oberschenkel bekannt.

Die Vorrichtung weist dabei einen bogenförmigen Körper auf, der an einem Ende in einen sich etwa rechtwinklig davon wegerstreckenden geradlinigen Abschnitt und dann in einen Handgriff übergeht.

Am gegenüberliegenden Ende ist am Bogen das Hüftgelenkimplantat montiert, und zwar so, dass sich der Kopf des Hüftgelenkimplantates von diesem Ende des Bogens weg erstreckt. Der stabförmige Abschnitt des Hüftgelenkimplantates, der in den Oberschenkel eingeschoben werden soll, erstreckt sich in Richtung des Handgriffes.

Dieser Zusammenbau wird an der zuvor präparierten Öffnung am Oberschenkelknochen angesetzt und durch eine Ziehbewegung in den Knochen eingeschoben.

Anschließend wird die Vorrichtung von dem Implantat gelöst und der von dem Oberschenkel vorstehende Kugelkopf des Hüftimplantates kann dann in die Pfanne des Hüftgelenkes eingesetzt werden.

Aus der WO 2005/122970 A1 ist ein Gerät zum Setzen oder Entfernen von Gelenken oder Gelenkpfannen bekannt. Diese Vorrichtung weist einen bogenförmigen Körper auf, der, zur Gewichtsersparnis, mit Materialaussparungen versehen ist. An einem Ende des Bogens ist ein Handgriff zum Manipulieren vorhanden, am gegenüberliegenden Ende ist ein Ansatz zum Anbringen einer Gelenkpfanne angeordnet.

Es ist daher Aufgabe der vorliegenden Erfindung, hier Abhilfe zu schaffen und ein Einbringinstrument zu schaffen, mit dem ein Verblockungselement einfach, sicher und ohne große Beschädigungsgefahr für den Sehnenersatz gelenknah eingebracht werden kann.

Erfindungsgemäß wird die Aufgabe durch ein Einbringinstrument zum Einschlagen eines Verblockungselementes in einen Bohrkanal zum Fixieren eines Sehnenersatzes in dem Bohrkanal gelöst, das einen bogenförmigen Körper aufweist, an dessen proximalem Ende ein Schlagkopf angeordnet ist, an dessen distalem Ende eine Anlage zum Anlegen eines Verblockungselementes angeordnet ist, und mit einer Haltevorrichtung zum Halten eines an die Anlage angelegten Verblockungselementes, wobei Schlagkopf und Anlage derart längs einer diese verbindenden Schlaglinie ausgerichtet sind, dass ein Schlagen auf den Schlagkopf ein Eintreiben des Verblockungselementes längs der Schlaglinie bewirkt, wobei der bogenförmige Körper an seinen beiden Enden Abschnitte aufweist, die sich etwa parallel zueinander und im Wesentlichen senkrecht zur Schlaglinie erstrecken.

Diese Maßnahmen haben nun die nachfolgenden zahlreichen Vorteile.

Das Verblockungselement wird nicht eingedreht, sondern eingeschlagen, und zwar längs des Bohrkanals. Dadurch ist prinzipiell ausgeschlossen, dass sich der Sehnenersatz dreht oder verdrillt und dabei beschädigt werden kann.

Durch Ausbilden eines bogenförmigen Körpers kann das distale Ende des Einbringinstrumentes mit dem daran angebrachten Verblockungselement in den intraartikulären Raum des Knies zwischen Tibia und Femur gebracht werden und das Verblockungselement kann dann beispielsweise in den tibialen Bohrkanal von intraartikulär nach extraartikulär gerichtet eingetrieben werden. Dabei reicht es für eine gelenknahe Fixierung aus, das Verblockungselement gerade über seine Länge in den entsprechenden Bohrkanal einzutreiben. Soll das Verblockungselement beispielsweise in den tibialen Bohrkanal eingetrieben werden, wird es soweit eingetrieben, dass dessen proximales Ende gerade mit dem Tibiaplateau abschließt. Die Eintreibrichtung von intraartikulär nach extraartikulär hat den Vorteil, dass der Sehnenersatz etwas in diese Richtung beim Einschlagen bewegt und somit gespannt wird. In umgekehrter Richtung wird der Sehnenersatz etwas in Richtung Kniegelenkmitte geschoben und somit entspannt werden, was unerwünscht ist.

Der gekrümmte Körper erstreckt sich abseits um den Bereich des Kniegelenkes herum, so dass das Einbringinstrument am Kniegelenk vorbei geführt werden kann. Im gekrümmten Bereich kann das Einbringinstrument zur Handhabung ergriffen werden. Der gekrümmte Bereich kann bogenförmig oder auch rechteckig verlaufend ausgebildet sein.

Durch die Konstruktion des gekrümmten Körpers und der Ausrichtung von Schlagkopf und Anlage des Verblockungselementes derart, dass sich die Schlaglinie in Richtung der Eintreibrichtung des Verblockungselementes erstreckt, kann das Verblockungselement gezielt in den entsprechenden Bohrkanal in dessen Längsrichtung eingetrieben werden. Die von dem Einschlagwerkzeug auf den Schlagkopf einwirkende Schlagkraft wird über den bogenförmigen Körper auf die Anlage am distalen Ende übertragen, und zwar in Richtung der diese beiden Endpunkte verbindenden Schlaglinie, die sich in Richtung der Längserstreckung des Verblockungselementes und auch in Richtung der Längserstreckung des entsprechenden Knochenkanals erstreckt.

Das heißt, das distale Ende mit dem daran angebrachten Verblockungselement kann ganz gezielt an die intraartikuläre Mündung des jeweiligen Bohrkanals angesetzt und ausgerichtet werden, und dann durch Schlagen auf den weit abseits davon gelegenen Schlagkopf sicher und zielgerichtet eingetrieben werden. Über den bogenförmigen Körper kann das Einbringinstrument außerdem gehalten werden.

Durch Vorsehen der Haltevorrichtung kann ein Verblockungselement an das Einbringinstrument angebracht werden, so dass dieses nicht von diesem herab fällt. Dieser Zusammenbau kann dann zielgerichtet und richtungsorientiert an dem Bohrkanal angesetzt und das Verblockungselement eingetrieben werden. Die Haltevorrichtung wird anschließend gelöst, so dass das Einbringinstrument vom eingeschlagenen Verblockungselement abgenommen werden kann.

Die parallelen Enden des bogenförmigen Körpers haben den Vorteil, dass mit dem geradlinigen distalen Endabschnitt gezielt in den Gelenkinnenraum eingefahren werden kann, um das Verblockungselement an der intraartikulären Mündung des Bohrkanals einzusetzen. Der entsprechend andere parallele Abschnitt gibt dem Arzt einen Hinweis, in welcher Richtung der bereits intraartikulär eingeschobene distale Abschnitt ausgerichtet ist, so dass er die Schlagrichtung zutreffend wählen kann, auch wenn ihm die intraartikuläre Einsicht erschwert ist.

Insgesamt gesehen kann mit einem sehr stabil ausgebildeten Einbringinstrument, das dennoch einfach handhabbar ist, auf einfache Weise ein Verblockungselement in den Zwischenraum zwischen Bohrkanalwand und Sehnenersatz durch wenige Einschlagvorgänge zielgerichtet, lagegerecht und schonend eingebracht werden.

Somit wird die Aufgabe vollkommen gelöst.

In einer weiteren Ausgestaltung der Erfindung ist der bogenförmige Körper derart ausgebildet, dass er von einer menschlichen Hand ergreifbar ist.

Diese Maßnahme hat den Vorteil, dass das Einbringinstrument bzw. der Zusammenbau aus Einbringinstrument und daran angebrachtem Verblockungselement für die Handhabungsperson einfach handhabbar ist, indem dieser den bogenförmigen Körper mit einer menschlichen Hand ergreift. Dabei bleiben die beiden endseitigen Stellen frei. Das heißt zum einen, die distale Endstelle, an der das Verblockungselement angebracht ist, so dass die Handhabungsperson visuell den Ansatzpunkt an der Mündung des Bohrkanals suchen kann. Das andere Ende liegt ebenfalls frei, so dass dann darauf mit dem Eintreiber, beispielsweise einem Hammer, geschlagen werden kann, ohne dass die Gefahr besteht, dass die Hand der Handhabungsperson verletzt wird.

In einer weiteren Ausgestaltung der Erfindung ist der bogenförmige Körper C-förmig oder rechteckig ausgebildet.

Es hat sich herausgestellt, dass diese Geometrien besonders günstig sind, da sie sehr große Freiheitsgrade der Handhabung erlauben und ergonomisch über eine Hand ergreifbar sind.

In einer weiteren Ausgestaltung der Erfindung sind im bogenförmigen Körper Materialaussparungen zur Gewichtsreduzierung vorgenommen.

Diese Maßnahme hat den Vorteil, dass der bogenförmige Körper an sich relativ voluminös ausgebildet sein kann, insbesondere in dem gekrümmten Bereich, in dem er von einer menschlichen Hand erfasst werden kann, so dass dieser großflächig in der Hand liegt. Durch die Materialaussparungen ist aber das Gewicht reduziert, so dass die Handhabungsperson das Einbringinstrument einfach handhaben kann. Es wird selbstverständlich dafür Sorge getragen, dass trotz der Materialaussparungen eine ausreichende Stabilität besteht, so dass sich beim Einschlagen der gebogene Körper nicht undefiniert verformt, sondern sich die Einschlagkraft exakt längs der Schlaglinie vom Schlagkopf über den gebogenen Körper umgeleitet in Richtung Anlage fortsetzt.

In einer weiteren Ausgestaltung der Erfindung ist der Schlagkopf nagelkopfartig ausgebildet.

Diese Maßnahme hat den Vorteil, dass dieses Schlagende mit einem Eintreiber, beispielsweise mit einem Hammer, sehr gut getroffen werden kann.

In einer weiteren Ausgestaltung der Erfindung weist die Anlage eine Anlagefläche auf, an die ein proximales Ende des Verblockungselementes anlegbar ist.

Diese Maßnahme hat den Vorteil, dass dadurch das Verblockungselement exakt ausgerichtet und schon in gewissem Maße fixiert an die Anlage angelegt werden kann.

In einer weiteren Ausgestaltung der Erfindung erstreckt sich eine Längsachse eines an die Anlage angelegten Verblockungselementes in der Schlaglinie.

Diese Maßnahme hat den Vorteil, dass dadurch besonders sanft ein länglicher Körper eines Verblockungselementes in den Bohrkanal in den Zwischenraum zwischen Innenwand und Sehnenersatz eingetrieben werden kann.

In einer weiteren Ausgestaltung der Erfindung ist am distalen Ende des bogenförmigen Körpers ein Vorsprung vorhanden, mit dem das Verblockungselement zum Versenken in dem tibialen Bohrkanal nachschlagbar ist.

Diese Maßnahme hat den erheblichen Vorteil, dass zunächst einmal das Verblockungselement soweit eingeschlagen werden kann, bis der distale Endabschnitt des Einbringinstruments mit dem Tibiaplateau oder den entsprechenden Kondülenflächen des Femurs fluchtet. Die Verbindung mit dem bereits schon weit eingeschlagenen Verblockungselement kann dann gelöst werden, der von dem Endabschnitt vorspringende Vorsprung kann dann auf den Kopf bzw. das intraartikuläre Ende des Verblockungselementes angesetzt werden und noch etwas nachgeschlagen werden, so dass sichergestellt ist, dass das intraartikuläre Ende des Verblockungselementes auf jeden Fall nicht über die Knocheninnenflächen vorsteht.

Dadurch kann eine besonders exakte, lagegerechte und gelenknahe Fixierung des Sehnenersatzes bewerkstelligt werden kann.

In einer weiteren Ausgestaltung der Erfindung weist die Haltevorrichtung eine Fixiervorrichtung für einen Faden des Verblockungselementes auf.

Diese Maßnahme hat den Vorteil, dass ein vom Verblockungselement vorstehender Faden über diese Fixiervorrichtung fixiert werden kann, so dass dann dadurch indirekt das Verblockungselement an der Anlage fixiert ist.

In einer weiteren Ausgestaltung der Erfindung ist die Fixiervorrichtung in einem Abstand zur Anlage angeordnet und der Faden ist in einer Führung im Bereich der Anlage aufgenommen.

Diese Maßnahme hat den Vorteil, dass der Faden durch die Führung ganz gezielt zu der Fixiervorrichtung geführt wird und dort von dieser fixiert wird.

Dadurch, dass diese abseits von der Anlage angeordnet ist, liegt diese abseits des Kniegelenkes bzw. des Operationsortes und stört den Einbring- oder Einschlagvorgang nicht bzw. kann unabhängig davon bedient werden.

Der Faden ist zwischen der Führung und der Fixiervorrichtung praktisch an zwei Stellen geführt und gespannt, was für eine besonders sichere und feste lagegerechte Halterung des Verblockungselementes an dem Instrument sorgt. Besonders einfach ist eine Fixierung durch eine Fixierschraube zu bewerkstelligen.

In einer weiteren Ausgestaltung der Erfindung ist die Führung als zum distalen Ende des bogenförmigen Körpers offener Schlitz ausgebildet, in den der Faden einlegbar ist.

Diese Maßnahme hat den Vorteil, dass das Anbringen und Fixieren des Verblockungselementes bzw. des Fadens besonders einfach durchzuführen ist.

Dabei wird so vorgegangen, dass der von dem Verblockungselement proximalseitig vorstehende Faden in den Schlitz eingeschoben wird, durch Ziehen an dem Faden wird dann das proximale Ende des Verblockungselementes fest an die Anlagefläche herangezogen. Der so gespannte Faden wird dann ein- oder zweimal um die Fixierschraube gedreht und durch diese fixiert.

Dadurch ist der Zusammenbau aus Einbringinstrument und daran fixiertem Verblockungselement gut handhabbar.

Dieses Fixieren kann schon vor dem eigentlichen Eingriff durch eine Hilfsperson bewerkstelligt werden, so dass bei dem eigentlichen operativen Eingriff der Operateur nur noch diesen Zusammenbau ergreifen, ansetzen und das Verblockungselement einschlagen muss.

Dies führt zu einer besonders ökonomischen Handhabung des Einbringelementes und ist einem schonenden Rekonstruktionsvorgang zuträglich.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: perspektivisch eine Ansicht eines erfindungsgemäßen Einbringinstrumentes,
- Fig. 2: eine Seitenansicht des Einbringinstrumentes von Fig. 1,
- Fig. 3: eine ausschnittweise Darstellung des distalen Endabschnittes, wobei an diesen ein Verblockungselement angebracht und fixiert ist,

- Fig. 4: eine der Darstellung von Fig. 3 vergleichbare perspektivische Ansicht eines distalen Endabschnittes eines erfindungsgemäßen Einbringinstrumentes, und
- Fig. 5: die Handhabung eines Einbringinstrumentes bei der Verblockung eines Sehnenersatzes in einem tibialen Bohrkanal.

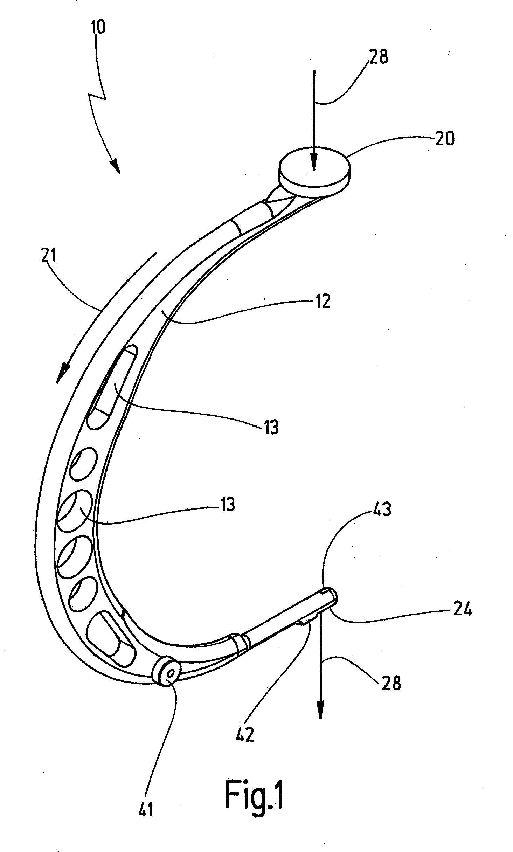

Ein in den Fig. 1 bis 5 dargestelltes erfindungsgemäßes Einbringinstrument ist in seiner Gesamtheit mit der Bezugsziffer 10 bezeichnet.

Das Einbringinstrument 10 weist einen bogenförmigen Körper 12 auf, dessen Krümmung etwa C-förmig ist. In anderen Ausführungen ist der Körper rechteckig geformt.

Der bogenförmige, etwa halbkreisförmige Körper 12 endet in einem etwa geradlinig verlaufenden proximalen Endabschnitt 14 bzw. in einem entsprechend geradlinig verlaufenden Endabschnitt 16.

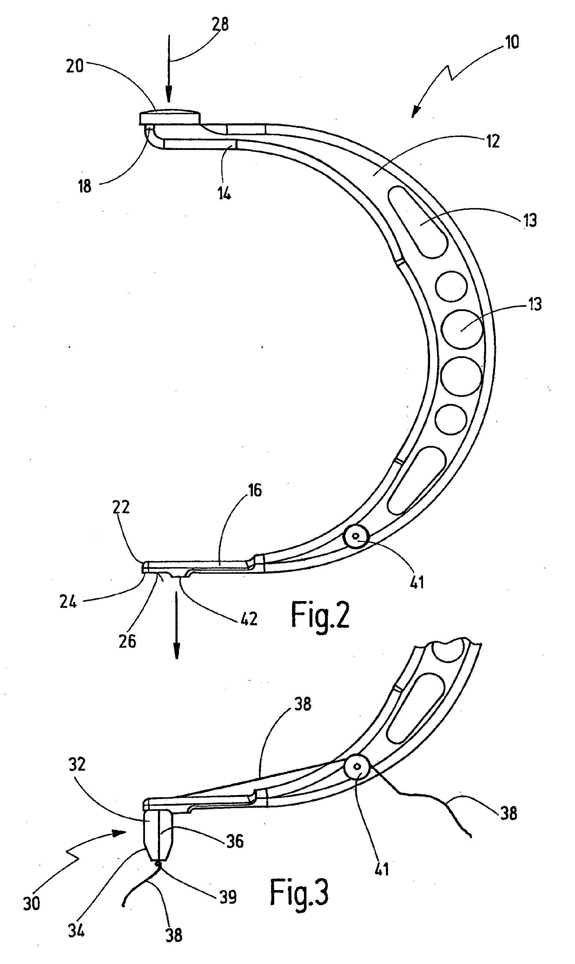

Wie insbesondere aus Fig. 2 zu erkennen, verlaufen die proximalen Endabschnitte 14 und 16 etwa parallel zueinander und enden auf etwa gleicher Höhe.

Das proximale Ende 18 des proximalen Endabschnittes 14 ist mit einem äußeren Schlagkopf 20 versehen, der etwa nagelkopfartig ausgebildet ist. Es ist somit möglich, mit einem Schlagwerkzeug, beispielsweise einem Hammer, auf den Schlagkopf 20 einzuschlagen.

Der distale Endabschnitt 16 weist im Bereich seines distalen Endes 22 eine zur Außenseite gerichtete Anlage 24 auf, die eine Anlagefläche 26 aufweist.

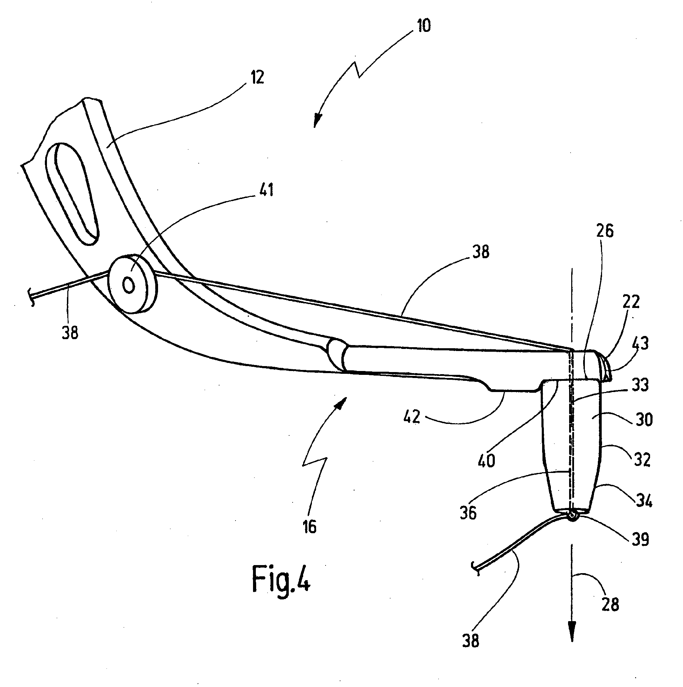

Wie das aus den Darstellungen von Fig. 3 und 4 ersichtlich ist, dient die Anlage 24 bzw. die Anlagefläche 26 dazu, dass an diese ein proximales Ende eines Verblockungselements 30 angelegt und auch fixiert werden kann.

Das Verblockungselement 30 weist einen etwa zylindrischen Körper 32 auf, der distalseitig eine Verjüngung 34 aufweist.

Längs seiner mittigen Längsachse 33 erstreckt sich eine Bohrung 36, durch die ein Faden 38 hindurchgeschoben ist.

Der Faden 38 ist jeweils an den Austrittsstellen aus der Bohrung 36 mit einem Knoten 39 versehen. Das vom proximalen Ende des Verblockungselementes 30 vorstehende Ende des Fadens 38 wird um eine Fixiervorrichtung in Form einer Fixierschraube 41 einer Haltevorrichtung gelegt, und zwar in einem gespannten Zustand, wie das insbesondere in Fig. 3 und in Fig. 4 ersichtlich ist.

Dabei liegt das proximale Ende 40 des Verblockungselementes 30 flächig und passend an der Anlagefläche 26 der Anlage 24 an. Wie aus Fig. 1 und insbesondere aus Fig. 4 ersichtlich ist, ist von dem distalen Ende 22 her ein Schlitz 43 in den bogenförmigen Körper 12 eingeschnitten, über den der Faden 38 des Verblockungselementes 30 eingeschoben werden kann. Das innere Ende des Schlitzes 43 endet etwa auf Höhe der Mittellängsachse 33 des Verblockungselements 30.

Durch Ziehen an dem Faden 38 in Richtung der Fixierschraube 41 wird somit das Verblockungselement 30 etwa rechtwinklig nach außen von dem distalen Endabschnitt 16 abstehend an die Anlagefläche 26 angelegt.

Durch Zudrehen der Fixierschraube 41 ist dann das Verblockungselement 30 fest an dem distalen Ende 22 gehalten. Diese Bauelemente, also Anlage 24, Schlitz 43, Faden 38 und Fixierschraube 41 bilden somit die Haltevorrichtung für das Verblockungselement 30. Die Mittellängsachse 33 des Verblockungselementes 30 ist so ausgerichtet, dass diese in einer Schlaglinie 28 liegt.

Die Schlaglinie 28 ist als eine gedachte Verbindungslinie anzusehen, die den Schlagkopf 20 mit der Anlage 24 verbindet.

Wird somit mit einem Schlagwerkzeug, beispielsweise mit einem Hammer, auf den Schlagkopf 20 geschlagen, und zwar in Richtung der Schlaglinie 28, werden die einwirkenden Kräfte über den bogenförmigen Körper 12 auf das distale Ende 22 übertragen, wie das in Fig. 1 durch einen Pfeil 21 dargestellt ist und können von dem Verblockungselement 30, wenn es dann montiert ist, in Richtung dessen Längsachse 33 bzw. eben der Schlaglinie 28 linear in Schlagrichtung fortgeführt werden.

Wie aus Fig. 1 und insbesondere Fig. 4 zu erkennen, ist neben der Anlage 24 ein Vorsprung 42 vorhanden, dessen Sinn und Zweck nachfolgend in Zusammenhang mit der Handhabung des Einbringinstrumentes 10 näher beschrieben wird.

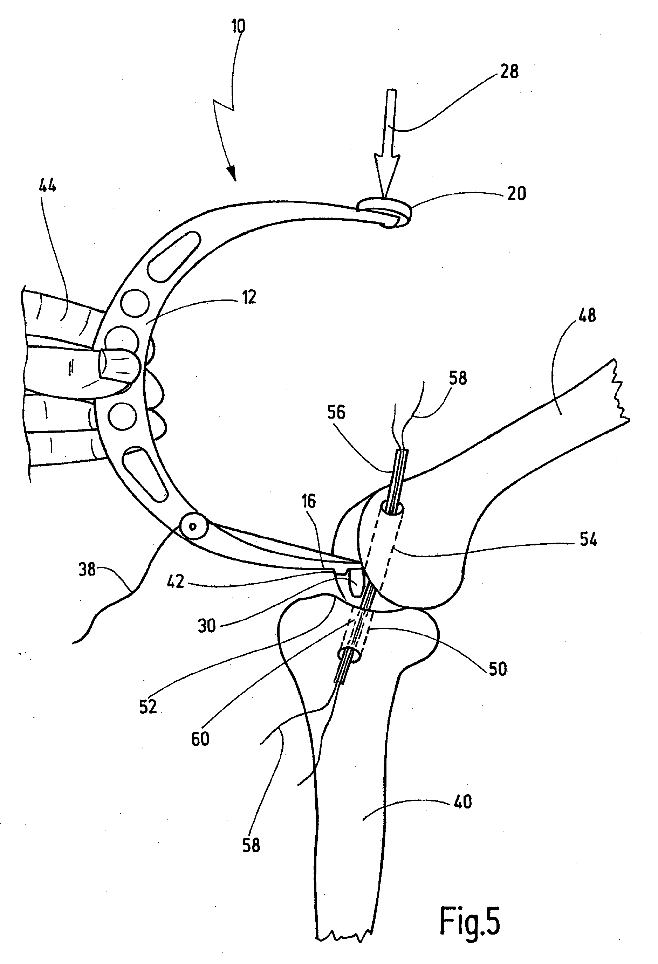

Eine solche Handhabung soll in Zusammenhang mit Fig. 5 beschrieben werden.

Dazu wird das Einbringinstrument 10, wie zuvor in Zusammenhang mit Fig. 1 beschrieben, vorbereitet, d.h. es wird ein Verblockungselement 30 an die Anlage 24 angesetzt und entsprechend fixiert.

In Fig. 5 ist ein Kniegelenk eines menschlichen Knies dargestellt, wobei hier lediglich die Tibia 46 (der Unterschenkelknochen) und der Femur 48 (der Oberschenkelknochen) dargestellt ist, die Kniescheibe wird dabei seitlich verschoben, so dass in den intraartikulären Zwischenraum ein Zugang geschaffen ist.

Zuvor wurde in die Tibia 46 von der Außenseite her ein tibialer Bohrkanal 50 eingebracht, der auf Höhe des sogenannten Tibiaplateaus 52 mündet.

Dieser Kanal ist als femoraler Bohrkanal 54 im Femur 48 bis zu dessen Außenseite fortgesetzt.

In die beiden Bohrkanäle 50 und 54 ist bereits ein Sehnenersatz 56 eingeschoben, wobei zu dessen Handhabung endseitige Fäden 58 vorgesehen sind.

Der Sehnenersatz 56 ist üblicherweise kürzer und ragt nicht beidseits über die Außenseite der Bohrkanäle 50 und 54 hinaus, dies ist hier nur zu demonstrativen Zwecken so dargestellt.

Der Sehnenersatz 56 kann ein künstlicher Sehnenersatz sein oder ein natürliche Sehne des Patienten, beispielsweise die Semitendonosussehne.

Der Sehnenersatz 56 muss nunmehr fixiert werden.

Zur Fixierung des im tibialen Bohrkanal 50 eingeschobenen Abschnittes des Sehnenersatzes 56 ist das Verblockungselement 30 vorgesehen.

Wie aus Fig. 5 zu erkennen, wird das Einbringinstrument 10 im Bereich dessen bogenförmigen Körpers 12 mit einer menschlichen Hand 44 ergriffen und dessen distaler Endabschnitt 16 wird mit dem Verblockungselement 30 in den intraartikulären Zwischenraum des Knies eingeführt, und zwar derart, dass das distale Ende des Verblockungselements 30 in Richtung der Mündung des tibialen Bohrkanals 50 ausgerichtet ist. Dabei wird der Zwischenraum 60 zwischen der Innenseite des tibialen Bohrkanals 50 und dem darin eingelegten Abschnitt des Sehnenersatzes 56 angezielt. Durch die Verjüngung 34 am Verblockungselement 30 ist dieser Ansatz etwas erleichtert. Die parallele Ausgestaltung der geradlinigen Enden des bogenförmigen Körpers 12 gibt dem Operateur eine Hilfe oder einen Hinweis auf die Ausrichtung des Verblockungselementes 30, auch wenn er dies selbst kaum oder gar nicht mehr erblicken kann. Das verhindert ein "schräges" Eintreiben des Verblockungselementes 30, so dass weder der Sehnenersatz 56 noch die Bohrung 54 dadurch beeinträchtigt werden.

Das Einbringinstrument 10 wird nunmehr so ausgerichtet, dass die Schlaglinie 28, also die Kraftübertragungslinie, sich in Richtung des Bohrkanals 50 ausrichtet.

In der Darstellung von Fig. 5 wird die Handhabungsperson den Schlagkopf 20 noch etwas nach rechts verschwenken, bis die Längsachse des Verblockungselementes 30 etwa in Ausrichtung mit der Längsachse des tibialen Bohrkanals 50 ist.

Dann wird mit einem Schlagwerkzeug, beispielsweise mit einem Hammer, auf den Schlagkopf 20, wie das durch den Pfeil 21 angedeutet ist, geschlagen und dabei wird das Verblockungselement 30 sanft in den Zwischenraum 60 zwischen der Innenseite des tibialen Bohrkanals 50 und dem darin aufgenommenen Sehnenersatz 56 eingebracht. Es ist zu erkennen, dass dies ohne Verdrillen oder Verletzungsgefahr für den Sehnenersatz 56 bewerkstelligt werden kann.

Das Einschlagen kann so weit betrieben werden, bis die dem Tibiaplateau 52 zugewandte Unterseite des bogenförmigen Körpers 12 diese erreicht.

Soll das Verblockungselement 30 dann noch etwas weiter ein- oder nachgeschlagen werden, kann die Fixierschraube 41 gelöst werden, so dass das Einbringinstrument 10 von dem bereits im tibialen Bohrkanal 50 steckenden Verblockungselement 30 abgehoben werden kann.

Das Einbringinstrument 10 kann nun so über das proximale Ende des Verblockungselementes 30 gebracht werden, dass genau der Vorsprung 42 auf diesem Ende sitzt. Durch ein nochmaliges Schlagen kann dann das Verblockungselement 30 so weit in den tibialen Bohrkanal 50 eingeschlagen oder eingesenkt werden, bis dieses nicht mehr über diesen hinausschaut.

Dadurch ist dann eine optimale gelenknahe Lage des Verblockungselements 30 erzielt.

Das Einbringinstrument 10 wird dann vom Gelenk abgezogen, der Faden 38 abgeschnitten und dann wird der Eingriff weitergeführt.

Soll der Sehnenersatz im Femur 48 entsprechend verblockt werden, kann an das Einbringinstrument 10 ein weiteres Verblockungselement 30 angesetzt und dann erneut intraartikulär das Einbringinstrument 10 dann um 180° nach unten verdreht angesetzt werden.

Bei manchen Operationstechniken ist es aber üblich, die bis zur Außenseite reichenden Fäden 58 an der Außenseite des Femurs 48, beispielsweise durch einen sogenannten Befestigungsknopf, zu befestigen.

Dies richtet sich aber nach der vom Operateur gewünschten Operationstechnik.

Nach einem Eingriff kann das Einbringinstrument 10 einfach gereinigt, desinfiziert und sterilisiert, und dann für einen neuen Einsatz vorbereitet werden.

Für eine ausreichende Stabilität ist der Körper 12 aus Medizinerstahl hergestellt, die C-förmige Geometrie sorgt für die ausreichende Stabilität, die Materialaussparungen für ein angenehmes Gewicht.

Im vorangegangenen Ausführungsbeispiel wurde die Haltevorrichtung mit der Fixierschaube und dem Faden beschrieben, es ist auch möglich, das distale Ende des Verblockungselements und die Anlagefläche 26 so zu konturieren, dass das Verblockungselement 30 fest angesetzt oder angeklipst oder verrastet werden kann. Die Verrastung kann so gelöst werden, dass das bereits eingeschlagene Verblockungselement ohne zu verdrehen in dem entsprechenden Bohrkanal verbleibt, das Einbringinstrument 10 aber beispielsweise durch Verkippen oder Verdrehen von dem Verblockungselement 30 abgenommen werden kann.

## Patentansprüche

1. Einbringinstrument zum Einschlagen eines Verblockungselements (30) in einen Bohrkanal (50) zum Fixieren eines Sehnenersatzes (56) in dem Bohrkanal (50) bei einer Rekonstruktion eines Kreuzbandes eines Kniegelenkes, mit einem bogenförmigen Körper (12), an dessen proximalem Ende (18) ein Schlagkopf (20) angeordnet ist, und an dessen distalem Ende (22) eine Anlage (24) zum Anlegen eines Verblockungselementes (30) angeordnet ist und mit einer Haltevorrichtung (41, 43) zum Halten eines an die Anlage (24) angelegten Verblockungselementes (30), wobei Schlagkopf (20) und Anlage (24) derart längs einer diese verbindenden Schlaglinie (28) ausgerichtet sind, dass ein Schlagen auf den Schlagkopf (20) ein Eintreiben des Verblockungselementes (30) längs der Schlaglinie (28) bewirkt,
**dadurch gekennzeichnet,**
**dass** der bogenförmige Körper (12) an seinen beiden Enden (18, 22) Abschnitte (14, 16) aufweist, die sich etwa parallel zueinander und im Wesentlichen senkrecht zur Schlaglinie (28) erstrecken.

2. Einbringinstrument nach Anspruch 1, **dadurch gekennzeichnet, dass** der bogenförmige Körper (12) derart ausgebildet ist, dass er von einer menschlichen Hand (44) ergreifbar ist.

3. Einbringinstrument nach Anspruch 1 oder 2, **dadurch gekennzeichnet, dass** der bogenförmige Körper (12) C-förmig oder rechteckig gebogen ist.

4. Einbringinstrument nach einem der Ansprüche 1 bis 3, **dadurch gekennzeichnet, dass** im bogenförmigen Körper (12) Materialaussparungen (13) zur Gewichtsreduzierung vorgenommen sind.

5. Einbringinstrument nach einem der Ansprüche 1 bis 4, **dadurch gekennzeichnet, dass** der Schlagkopf (20) nagelkopfartig ausgebildet ist.

6. Einbringinstrument nach einem der Ansprüche 1 bis 5, **dadurch gekennzeichnet, dass** die Anlage (24) eine Anlagefläche (26) aufweist, an die ein proximales Ende (40) eines Verblockungselementes (30) anlegbar ist.

7. Einbringinstrument nach einem der Ansprüche 1 bis 6, **dadurch gekennzeichnet, dass** sich eine Längsachse (33) eines an die Anlage (24) angelegten Verblockungselementes (30) in der Schlaglinie (28) erstreckt.

8. Einbringinstrument nach einem der Ansprüche 1 bis 7, **dadurch gekennzeichnet, dass** am distalen Ende (22) des bogenförmigen Körpers (12) ein Vorsprung (42) vorhanden ist, mit dem das Veiblockungselement (30) zum Versenken in dem Bohrkanal (50) nachschlagbar ist.

9. Einbringinstrument nach einem der Ansprüche 1 bis 8, **dadurch gekennzeichnet, dass** die Haltevorrichtung eine Fixierschraube (41) für einen Faden (38) des Verblockungselementes (30) aufweist.

10. Einbringinstrument nach Anspruch 9, **dadurch gekennzeichnet, dass** die Fixierschraube (41) in einem Abstand zur Anlage (24) angeordnet ist, und dass der Faden (38) in einer Führung im Bereich der Anlage (24) aufgenommen ist.

11. Einbringinstrument nach Anspruch 10, **dadurch gekennzeichnet, dass** die Führung als zum distalen Ende (22) des bogenförmigen Körpers (12) offener Schlitz (43) ausgebildet ist, in den der Faden (38) einlegbar ist.

## Claims

1. Insertion instrument for hammering a blocking element (30) into a drilled channel (50) for fixing a tendon replacement (56) in the drilled channel (50) in a reconstruction of a cruciate ligament of a knee joint, with an arc-shaped body (12), at the proximal end (18) of which a striking head (20) is arranged, and at the distal end (22) of which an abutment (24) is arranged for placing a blocking element (30), and with a holding device (41, 43) for holding a blocking element (30) placed on the abutment (24), wherein striking head (20) and abutment (24) are oriented along a strike line (28) connecting them, in such a way that a strike on the striking head (20) causes the blocking element (30) to be driven in along the strike line (28), **characterized in that** the arcshaped body (12) has, at its two ends (18, 22), portions (14, 16) which extend approximately parallel to each other and substantially perpendicularly with respect to the strike line (28).

2. Insertion instrument according to Claim 1, **characterized in that** the arc-shaped body (12) is designed in such a way that it can be gripped by a human hand (44).

3. Insertion instrument according to Claim 1 or 2, **characterized in that** the arc-shaped body (12) is bent in a C-shape or rectangularly.

4. Insertion instrument according to one of Claims 1 to 3, **characterized in that** material cutouts (13) are made in the arc-shaped body (12) in order to reduce its weight.

5. Insertion instrument according to one of Claims 1 to 4, **characterized in that** the striking head (20) is designed like a nail head.

6. Insertion instrument according to one of Claims 1 to 5, **characterized in that** the abutment (24) has an abutment surface (26) onto which a proximal end (40) of a blocking element (30) can be placed.

7. Insertion instrument according to one of Claims 1 to 6, **characterized in that** a longitudinal axis (33) of a blocking element (30) placed on the abutment (24) extends along the strike line (28).

8. Insertion instrument according to one of Claims 1 to 7, **characterized in that** a projection (42) is present at the distal end (22) of the arc-shaped body (12), with which projection (42) the blocking element (30) can be restruck in order to embed it in the drilled channel (50).

9. Insertion instrument according to one of Claims 1 to 8, **characterized in that** the holding device has a fixing screw (41) for a thread (38) of the blocking element (30).

10. Insertion instrument according to Claim 9, **characterized in that** the fixing screw (41) is arranged at a distance from the abutment (24), and **in that** the thread (38) is received in a guide in the area of the abutment (24).

11. Insertion instrument according to Claim 10, **characterized in that** the guide is designed as a slit (43) which is open towards the distal end (22) of the arc-shaped body (12) and into which the thread (38) can be fitted.

## Revendications

1. Instrument d'introduction pour enfoncer un élément de blocage (30) dans un canal foré (50) afin de fixer un substitut de tendon (56) dans le canal foré (50) lors d'une reconstruction d'un tendon croisé d'une articulation de genoux, avec un corps (12) en forme d'arc au niveau de l'extrémité proximale (18) duquel une tête percutante (20) est disposée et au niveau de l'extrémité distale (22) duquel un élément d'appui (24) est disposé pour épauler un élément de blocage (30) et avec un dispositif d'arrêt (41, 43) servant à arrêter un élément de blocage (30) épaulé contre l'élément d'appui (24), la tête percutante (20) et l'élément d'appui (24) étant orientés de telle sorte le long d'une ligne de frappe (28) les reliant qu'une frappe sur la tête percutante (20) provoque l'enfoncement de l'élément de blocage (30) le long de la ligne de frappe (28), **caractérisé en ce que** le corps (12) en forme d'arc comporte au niveau de ses deux extrémités (18, 22) des sections (14, 16) s'étendant quelque peu parallèlement l'une par rapport à l'autre et s'étendant pour l'essentiel perpendiculairement à la ligne de frappe (28).

2. Instrument d'introduction selon la revendication 1, **caractérisé en ce que** le corps (12) en forme d'arc est réalisé de telle sorte qu'il peut être saisi par une main (44) humaine.

3. Instrument d'introduction selon la revendication 1 ou 2, **caractérisé en ce que** le corps (12) en forme d'arc est incurvé selon une forme de C ou à angle droit.

4. Instrument d'introduction selon l'une quelconque des revendications 1 à 3, **caractérisé en ce que** des évidements de matière (13) sont réalisés dans le corps (12) en forme d'arc pour en réduire le poids.

5. Instrument d'introduction selon l'une quelconque des revendications 1 à 4, **caractérisé en ce que** la tête percutante (20) prend une forme de tête de clou.

6. Instrument d'introduction selon l'une quelconque des revendications 1 à 5, **caractérisé en ce que** l'élément d'appui (24) comporte une surface d'élément d'appui (26) contre laquelle une extrémité proximale (40) d'un élément de blocage (30) peut être épaulée.

7. Instrument d'introduction selon l'une quelconque des revendications 1 à 6, **caractérisé en ce qu'**un axe longitudinal (33) d'un élément de blocage (30) épaulé contre l'élément d'appui (24) s'étend dans la ligne de frappe (28).

8. Instrument d'introduction selon l'une quelconque des revendications 1 à 7, **caractérisé en ce qu'**une saillie (42) avec laquelle l'élément de blocage (30) peut être percuté dans un second temps est présente au niveau de l'extrémité distale (22) du corps (12) en forme d'arc pour le faire descendre dans le canal foré (50).

9. Instrument d'introduction selon l'une quelconque des revendications 1 à 8, **caractérisé en ce que** le dispositif d'arrêt est une vis de fixation (41) pour un fil (38) de l'élément de blocage (30).

10. Instrument d'introduction selon la revendication 9, **caractérisé en ce que** la vis de fixation (41) est disposée à une certaine distance de l'élément d'appui (24) et que le fil (38) est logé dans un guide prévu dans la région de l'élément d'appui (24).

11. Instrument d'introduction selon la revendication 10, **caractérisé en ce que** le guide prend la forme d'une fente (43) ouverte en direction de l'extrémité distale (22) du corps (12) en forme d'arc et dans laquelle le fil (38) peut être introduit.
